# EUROPEAN PATENT APPLICATION

(11) **EP 3 251 657 A1**
(43) Date of publication of application: **06.12.2017**
(21) Application number: 17173510.3
(22) Date of filing: 30.05.2017
(51) Int. Cl.: A61K 9/00, A61K 47/10, A61K 9/12, A61K 31/573

(54) **TOPICAL AQUEOUS SPRAY COMPOSITIONS OF HALOBETASOL**

(30) Priority: 30.05.2016 IN 201611018448
(71) Applicant: Sun Pharmaceutical Industries Limited, Mumbai, Maharashtra 400 063 (IN)
(72) Inventor: RANA, Anil, 160047 Chandigarh (IN); KESHRI, Lav, 226020 Lucknow (IN); BEDI, Simrata, 122001 Gurgaon (IN); KOCHHAR, Ravi, 122003 Gurgaon (IN); DESHMUKH, Subodh, Quaker Hill, CT Connecticut 06375 (US)
(74) Representative: Cronin, Brian Harold John

(57) **Abstract**

The present invention relates to topical aqueous spray compositions of halobetasol. The topical aqueous spray compositions may be in the form of solutions or emulsions. It also relates to processes for the preparation of the topical aqueous spray compositions. It further relates to a method of treating topical skin conditions by administering said topical aqueous spray compositions.

## Description

### Field of the Invention

The present invention relates to topical aqueous spray compositions of halobetasol. The topical aqueous spray compositions may be in the form of solutions or emulsions. It also relates to processes for the preparation of the topical aqueous spray compositions. It further relates to a method of treating topical skin conditions by administering said topical aqueous spray compositions.

### Background of the Invention

Halobetasol, a high potency corticosteroid commercially available as a cream, an ointment, and a lotion under the trade name Ultravate^{®}, has been used for the relief of inflammatory and pruritic manifestations of corticosteroid-responsive dermatoses. These compositions are applied to the affected skin by taking an amount thereof on a fingertip and then gently rubbing the composition into the skin. These compositions are not suitable where touching of sore or otherwise adversely affected skin should be minimized so that it does not cause any unnecessary additional discomfort. The high viscosity of these compositions makes physical application difficult when attempting to apply to large areas of skin.

Pharmaceutical sprays exhibit numerous advantages over these topical delivery systems. These advantages include easy delivery of the formulation to areas of the body that are difficult to treat, the possibility of better controlling the dose, the unlike liness of contamination during use, and convenience when applying to a large skin area,all of whichresult in enhanced patient compliance.

PCT Publication No. WO2015/044857 discloses a topical spray composition of halobetasol comprising halobetasol, an emollient, a non-aqueous solvent, and a propellant.

PCT Publication No. WO2015/044879 discloses a propellant-free topical spray composition of halobetasol comprising halobetasol, an emollient, and a non-aqueous solvent.

The present invention provides alternate spray compositions of halobetasol which are aqueous based.These spray compositions are non-occlusive, non-irritant, and provide enhanced patient compliance.

### Summary of the Invention

The present invention provides topical aqueous spray compositions of halobetasol which are useful in treating skin conditions such as corticosteroid-responsive dermatoses, including psoriasis and atopic dermatitis. As improved hydration of these skin conditions is desirable, the aqueous-based spray compositions provide beneficial effects. Moreover, the topical aqueous spray compositions of the present invention are preservative-free. The compositions of the present invention may be applied without rubbing onto the area of application, which otherwise could cause irritation to the already inflamed skin. The topical aqueous spray compositions of the present invention may be in the form of solutions or emulsions. The present invention also relates to processes for the preparation of the topical aqueous spray compositions. It further relates to a method of treating topical skin conditions by administering said topical aqueous spray compositions.

### Detailed Description of the Invention

As used herein, the word "a" or "plurality" before a noun represents one or more of the particular noun. For the terms "for example," "such as," and grammatical equivalences thereof, the phrase "and without limitation" is understood to follow unless explicitly stated otherwise. As used herein, the term "about" is meant to account for variations due to experimental error. All measurements reported herein are understood to be modified by the term "about," whether or not the term is explicitly used, unless explicitly stated otherwise. As used herein, the singular forms "a," "an," and "the" include plural referents unless the context clearly dictates otherwise.

Unless otherwise defined, all technical and scientific terms used herein have the same meaning as commonly understood by one of ordinary skill in the art to which this invention belongs. Methods and materials are described herein for use in the present invention; other suitable methods and materials known in the art can also be used. The materials, methods, and examples are illustrative only and not intended to be limiting. All publications, patent applications, patents, sequences, database entries, and other references mentioned herein are incorporated by reference in their entirety. In case of conflict, the present specification, including definitions, will control.

A first aspect of the present invention provides a topical aqueous spray composition comprising from about 0.01% w/w to about 0.5% w/w of halobetasol based on the total weight of the composition, the composition being aqueous and is to be placed in and/or released from a dispensing system for spraying said composition.

According to one embodiment of the above aspect, the composition comprises about 0.05% w/w of halobetasol based on the total weight of the composition.

According to another embodiment of the above aspect, the halobetasol is halobetasol propionate.

According to another embodiment of the above aspect, the composition is a solution.

According to another embodiment of the above aspect, the composition comprises one or morewater-miscible co-solvents and water.

According to another embodiment of the above aspect, the water-miscible co-solvent is selected from the group consisting of alcohols and glycols including ethyl alcohol, isopropyl alcohol, propylene glycol, butanediol, pentanediol, hexanediol, triethylene glycol, tetraethylene glycol, dipropylene glycol, and dibutylene glycol; glycerin; dimethyl isosorbide; tetrahydrofurfuryl alcohol polyethylene glycol ether; N-methyl-2-pyrrolidone; 1-methyl-2-pyrrolidinone; dimethylsulfoxide; dimethylacetamide; lactic acid; glycolic acid; methylene chloride; methyl-ethyl-ketone; ethyl acetate; methylene dimethyl ether; polyethylene glycols such as polyethylene glycol 400; and mixtures thereof. In a preferred embodiment, the water-miscible co-solvent is ethyl alcohol.

According to another embodiment of the above aspect, the composition is an emulsion.

According to another embodiment of the above aspect, the composition comprises one or more water-immiscible co-solvents, an emulsifier, and water.

According to another embodiment of the above aspect, the water-immiscible co-solvent is selected from the group consisting of fatty acid esters including isopropyl myristate and isopropyl palmitate; light and heavy liquid paraffins; fatty acid triglycerides including mixtures of caprylic and capric triglycerides (*e.g*.,Crodamol™ GTCC, Miglyol^{®}, Captex™, Labrafac™, Lipophile WL); mixtures of mono-, di-, and/or triglycerides; waxes; hydrogenated vegetable oils; and mixtures thereof.

According to another embodiment of the above aspect, the emulsifier is selected from the group consisting of sucrose esters; polymers including poloxamers; polysorbates such as Tween^{®} 20, Tween^{®} 40, Tween^{®} 60, and Tween^{®} 80; polyoxyethylene fatty acid esters including Myrj^{®} 45, Myrj^{®} 49, and Myrj^{®} 59; poly(oxyethylene)alkylyl ethers including poly(oxyethylene)cetyl ether, poly(oxyethylene)palmityl ether, polyethylene oxide hexadecyl ethers such as Brij^{®} 58, and polyethylene glycol cetyl ethers such as Brij^{®} 38, Brij^{®} 52, Brij^{®} 56, and Brij^{®} W1; partial esters of sorbitol and sorbitol anhydrides including sorbitan monolaurate and sorbitan monolaurate-mono or diglycerides; alkyl isethionates; alkyl and alkyl ether sulfates and salts thereof; alkyl and alkyl ether phosphates and salts thereof; alkyl methyl taurates; soaps (*e.g.*, alkali metal salts, sodium salts, and potassium salts of fatty acids); alkylimino acetates; imino-dialkanoates and aminoalkanoates; imidazolinium and ammonium derivatives; betaines; sultaines; hydroxysultaines; alkyl sarcosinates; alkanoyl sarcosinates; sorbitan esters including Arlacel^{®} 20 and Arlacel^{®} 40; polyethylene glycol ethers of stearic acid including steareth-2, steareth-4, and steareth-6; polyethylene glycol ethers of stearyl alcohol (steareth-21); silicone emulsifying agents including dimethicone copolyols; polyoxyethylene sorbitan fatty acid esters; non-solid fatty substances at room temperature including sesame oil, sweet almond oil, apricot stone oil, sunflower oil, octoxyglyceryl palmitate (or 2-ethylhexyl glyceryl ether palmitate), octoxyglyceryl behenate (or 2-ethylhexyl glyceryl ether behenate), and dioctyl adipate; PEG-20 hexadecenyl succinate; PEG-15 stearyl ether; disodium cocoamphodiacetate; oxyethylenated glyceryl cocoate (7 EO); ricinoleic monoethanolamide monosulfosuccinate salts; oxyethylenated hydrogenated ricinoleic triglyceridesincluding Cremophor^{®} RH 60 (polyoxyl 60 hydrogenated castor oil)and Cremophor^{®} RH 40 (polyoxyl 40 hydrogenated castor oil); and mixtures thereof.

According to another embodiment of the above aspect, the composition may further comprisean emollient.

According to another embodiment of the above aspect, the composition may further comprise a propellant forming a pressurized system, wherein the pressurized composition may be delivered through a dispensing system comprising a container and a valve-actuator assembly. Alternatively, the composition may be a propellant-free composition forming a non-pressurized system, wherein the non-pressurized composition may be delivered through a dispensing system comprising a container and a pump-actuator assembly.

According to another embodiment of the above aspect, the valve-actuator assembly may comprise a metered valve or a continuous valve. In a preferred embodiment, the valve-actuator assembly comprises a metered valve.

According to another embodiment of the above aspect, the pump-actuator assembly comprises a metered pump.

According to another embodiment of the above aspect, the topical aqueous spray composition is stable.

A second aspect of the present invention provides a process for the preparation of a topical aqueous spray composition of halobetasol, wherein the process comprises the steps of:
(a) dissolving halobetasol in a water-miscible co-solvent to form a solution;
(b) mixing the solution of step (a) with water; and
(c) dispensing the solution of step (b) in a dispensing system.

According to one embodiment of the above aspect, the dispensing system may be further charged with a propellant.

A third aspect of the present invention provides a process for the preparation of a topical aqueous spray composition of halobetasol, wherein the process comprises the steps of:
(a) dissolving halobetasol in a water-immiscible co-solvent to form a solution;
(b) dissolving an emulsifier in water to form a solution;
(c) mixing the solution of step (a) with the solution of step (b) to form an emulsion; and
(d) dispensing the emulsion of step (c) in a dispensing system.

According to one embodiment of the above aspect, the dispensing system may be further charged with a propellant.

A fourth aspect of the present invention provides a method of treating a topical skin condition by administering the topical aqueous spray composition of halobetasol.

According to one embodiment of the above aspect, the skin condition is selected from the group consisting of one or more of corticosteroid-responsive dermatoses including psoriasis, atopic dermatitis, eczema, rosacea, acne vulgaris, pruritis, seborrhea, skin cancers, and inflammation.In particular, the condition is a corticosteroid-responsive dermatosis.

According to another embodiment of the above aspect, the method comprises coadministration of additional drug(s) used to treat the topical skin condition.

The topical aqueous spray compositions of the present invention may be in the form of a solution or an emulsion. Alternatively, the topical aqueous spray composition may be in the form of a suspension comprising halobetasol partially or completely dispersed in water using a suspending agent. The suspension may additionally include a wetting agent.

The term "topical," as used herein, refers to a composition meant for application to the skin, nail, or mucosal tissue.

The term "spray," as used herein, means to dispense the composition as a mass or jet of droplets from a dispensing system.

The term "stable," as used herein, means chemical stability, wherein not more than 5% w/w of total related substances are formed on storage at 40°C and 75% relative humidity or at 25°C and 60% relative humidity for a period of at least three months to the extent necessary for sale and use of composition.

The term "halobetasol," as used herein, includes halobetasol and its salts, polymorphs, hydrates, solvates, prodrugs, chelates, and complexes. The preferred ester of halobetasol is halobetasol propionate. The topical spray composition of the present invention comprises halobetasol in an amount of from about 0.01% w/w to about 0.5% w/w based on the total weight of the composition.In particular, halobetasol is present in an amount of about 0.05% w/w based on the total weight of the composition.

The term "co-solvent," as used herein, refers to the solvent used to dissolve halobetasol.The co-solvent can be a water-miscible co-solvent used to form the solution of the present invention. Alternatively, the co-solvent can be a water-immiscible co-solvent used to form the emulsion of the present invention. Suitable water-miscible co-solvents are selected from the group comprising alcohols and glycols such as ethyl alcohol, isopropyl alcohol, propylene glycol, butanediol, pentanediol, hexanediol, triethylene glycol, tetraethylene glycol, dipropylene glycol, and dibutylene glycol; glycerin; dimethyl isosorbide; tetrahydrofurfuryl alcohol polyethylene glycol ether; N-methyl-2-pyrrolidone; 1-methyl-2-pyrrolidinone; dimethylsulfoxide; dimethylacetamide; lactic acid; glycolic acid; methylene chloride; methyl-ethyl-ketone; ethyl acetate; methylene dimethyl ether; polyethylene glycols such as polyethylene glycol 400; and mixtures thereof. In particular, the water-miscible co-solvent is ethyl alcohol. Suitable water-immiscible co-solvents are selected from the group comprising fatty acid esters such as isopropyl myristate and isopropyl palmitate; light and heavy liquid paraffins; fatty acid triglycerides such as mixtures of caprylic and capric triglycerides (*e.g*.,Crodamol™ GTCC, Miglyol^{®}, Captex^{®}, Labrafac^{®} Lipophile WL); mixtures of mono-, di-, and/or triglycerides; waxes; hydrogenated vegetable oils; and mixtures thereof. The co-solvent is present in an amount of from about 5% w/w to about 70% w/w based on the total weight of the composition.

The term "emollient," as used herein, refers to a substance that helps to retain the skin's moisture and also helps to control the rate of evaporation and the tackiness of the composition. Additionally, emollients provide a softening or soothing effect on the skin surface. Suitable examples of emollients are selected from the group comprising fatty acid triglycerides such as mixtures of caprylic and capric triglycerides (*e.g*.,Crodamol™ GTCC, Miglyol^{®}, Captex^{®}, Labrafac^{®} Lipophile WL), palmitic triglyceride, oleic triglyceride, caprylic triglyceride, capric triglyceride, and linoleic triglyceride; fatty acid esters such as isopropyl myristate, isopropyl palmitate, dibutyl adipate, and dibutyl phthalate; polyhydric alcohols such as propylene glycol, butylene glycol, polyethylene glycols such as PEG 400, glycerol, and sorbitol; fatty acids such as oleic acid and stearic acid; oils such as mineral oil, lanolin oil, coconut oil, cocoa butter, olive oil, jojoba oil, and castor oil; cyclomethicone; hydrogenated lanolin; waxes; lecithin; and mixtures thereof. Preferably, the emollient of the present invention is selected from the group consisting of fatty acid triglycerides, fatty acid esters, and polyhydric alcohols.The emollient is present in an amount of from about 2% w/w to about 60% w/w based on the total weight of the composition.

The term "propellant," as used herein, refers to the substance that helps in propelling the composition out of the container. Suitable propellants are selected from the group comprising conventional, non-ozone depleting hydrocarbon propellants. These include propane; butane; isobutane;pentane; isopentane;cyclopropane; 1,1,1,2-tetrafluoroethane; 1,1,1,2,3,3,3-heptafluoropropane; 1,1-difluoroethane; 1,1,1,3,3,3- hexafluoropropane; dimethyl ether; fluorocarbon gas; liquefied petroleum gas; compressed gases such as nitrogen, carbon dioxide, and nitrous oxide;and mixtures thereof. The propellant is present in an amount of from about 3% w/w to about 85% w/w based on the total weight of the composition.

The term "emulsifier," as used herein, refers to a substance that acts as a stabilizer and prevents the composition from separating into two phases. The emulsifier used in the present invention may be a non-ionic, a cationic, an anionic, or a zwitterionic emulsifier. Suitable emulsifiers are selected from the group comprising sucrose esters; polymers such as poloxamers; polyysorbates such as Tween^{®} 20, Tween^{®} 40, Tween^{®} 60, and Tween^{®} 80; polyoxyethylene fatty acid esters such as Myrj^{®} 45, Myrj^{®} 49, and Myrj^{®} 59; poly(oxyethylene)alkylyl ethers such as poly(oxyethylene)cetyl ether, poly(oxyethylene)palmityl ether, polyethylene oxide hexadecyl etherssuch as Brij^{®} 58, and polyethylene glycol cetyl ethers such asBrij^{®}38, Brij^{®}52, Brij^{®}56, and Brij^{®}W1; partial esters of sorbitol and sorbitol anhydrides such as sorbitan monolaurate and sorbitan monolaurate-mono or diglycerides; alkyl isethionates; alkyl and alkyl ether sulfates and salts thereof; alkyl and alkyl ether phosphates and salts thereof; alkyl methyl taurates; soaps (e.g., alkali metal salts, sodium salts, and potassium salts of fatty acids); alkylimino acetates; imino-dialkanoates and aminoalkanoates; imidazolinium and ammonium derivatives; betaines; sultaines; hydroxysultaines; alkyl sarcosinates; alkanoyl sarcosinates, sorbitan esters such as Arlacel^{®} 20 and Arlacel^{®} 40;polyethylene glycol ethers of stearic acid such as steareth-2, steareth-4, and steareth-6; polyethylene glycol ethers of stearyl alcohol (steareth-21); silicone emulsifying agents such as dimethicone copolyols; polyoxyethylene sorbitan fatty acid esters; non-solid fatty substances at room temperature such as sesame oil, sweet almond oil, apricot stone oil, sunflower oil, octoxyglyceryl palmitate (or 2-ethylhexyl glyceryl ether palmitate), octoxyglyceryl behenate (or 2-ethylhexyl glyceryl ether behenate), and dioctyl adipate; PEG-20 hexadecenyl succinate; PEG-15 stearyl ether; disodium cocoamphodiacetate; oxyethylenated glyceryl cocoate (7 EO); ricinoleic monoethanolamide monosulfosuccinate salts; oxyethylenated hydrogenated ricinoleic triglycerides such as Cremophor^{®} RH 60 (polyoxyl 60 hydrogenated castor oil) and Cremophor^{®} RH 40 (polyoxyl 40 hydrogenated castor oil); and mixtures thereof.

The term "about," as used herein, refers to any value which lies within the range defined by a variation of up to ±10% of the value.

The emulsion of the present invention has two phases, *i.e.*, an oily phase and an aqueous phase. The oily phase comprises of a water-immiscible co-solvent and the aqueous phase comprises of water or a mixture of water with a water-miscible solvent. The emulsion may be an oil-in-water type of emulsion or a water-in-oil type of emulsion, wherein the average size of a droplet in a dispersed phase is less than 200 µm. Alternatively, the emulsion may be in the form of a nanoemulsion.

The topical aqueous spray composition of the present invention further comprises permeation enhancers, film-formers, plasticizers, antioxidants, pH-adjusting agents, viscosity modifying agents, and mixtures thereof.

The permeation enhancer is a substance used to enhance the penetration rate of halobetasol through the skin. Suitable permeation enhancers are selected from the group comprising lipophilic solvents such as dimethyl sulfoxide and dimethyl formamide; non-ionic and ionic surfactants such as polyoxyethylyene-sorbitan; fatty acid esters such as polysorbates; ethers of sugars; ethoxylated fatty alcohols; sodium lauryl sulfate; taurocholic acid; lecithin; Labrasol^{®}; fatty acid esters such as isopropyl myristate and isopropyl palmitate; fatty acids such as oleic acid and stearic acid; polyhydric alcohols such as propylene glycol and polyethylene glycol e.g., polyethylene glycol 400; Transcutol^{®}; essential oils such as menthol; and combinations thereof.

The film-former is a substance that forms a stable film on a topical surface when applied. Suitable film-formers are selected from the group comprising acrylic polymers or copolymers such as methacrylic acid copolymers; cellulose derivatives such as cellulose acetate, hydroxypropyl methyl cellulose, hydroxy ethyl cellulose, hydroxy propyl cellulose, methyl cellulose, and ethyl cellulose; polyvinyl acetate; polyvinyl alcohol; povidone; povidone vinyl acetate; and combinations thereof. These film-formers can partially dissolve on exposure to moisture from the skin or air, the dissolution resulting in the formation of a porous film. This porosity can be enhanced by including additional water-soluble additives. The water-soluble additive is preferably propylene glycol, sodium lauryl sulphate, poloxamers, polyoxyl 35 castor oil, polyoxyl 40 hydrogenated castor oil, cetomacrogol, polyethylene glycol, Transcutol^{®}, or combinations thereof.

The plasticizer is a substance that aids the composition in forming a flexible, adherent film on the skin. Suitable plasticizers are selected from the group comprising citric acid esters, dimethyl isosorbide, castor oil, propylene glycol, polyethylene glycol, glycerol, oleic acid, citric acid, phosphate esters, fatty acid esters, glycol derivatives, hydrocarbons and their derivatives, adipic acid, butanediol polyesters, diethyl phthalate, dibutyl phthalate, chlorinated paraffins, and combinations thereof.

Suitable antioxidants are selected from the group comprising butylated hydroxyl anisole, butylated hydroxy toluene, sodium metabisulfite, ascorbic acid, ascorbyl palmitate, thiourea, acetylcysteine, dithiothreitol, cysteine hydrochloride, propyl gallate, tocopherol, and combinations thereof.

Suitable pH-adjusting agents are selected from the group comprising pharmaceutically acceptable organic or inorganic acids such as citric acid, hydrochloric acid, and inorganic salts of weak acids; bases such as sodium hydroxide and tromethamine; inorganic oxides; and combinations thereof. The pH-adjusting agent is present in an amount of from about 0.001% w/w to about 7% w/w based on the total weight of the composition.

Suitable viscosity modifying agents are selected from the group comprising cellulose derivatives, polyvinylpyrrolidone, and the like.

Suitable suspending agents are selected from the group comprising cellulose derivatives such as co-processed spray dried forms of microcrystalline cellulose and carboxymethyl cellulose sodium, hydroxypropyl cellulose, ethyl cellulose, hydroxyethyl cellulose, hydroxypropylmethyl cellulose, methylcellulose, carboxymethyl cellulose and its salts/derivatives, and microcrystalline cellulose; carbomers; gums such as locust bean gum, xanthan gum, tragacanth gum, arabinogalactan gum, agar gum, gellan gum, guar gum, apricot gum, karaya gum, sterculia gum, acacia gum, gum arabic, and carrageenan; pectin; dextran; gelatin; polyethylene glycols; polyvinyl compounds such as polyvinyl acetate, polyvinyl alcohol, and polyvinyl pyrrolidone; sugar alcohols such as xylitol and mannitol; colloidal silica; and mixtures thereof. Co-processed spray dried forms of microcrystalline cellulose and carboxymethyl cellulose sodium have been marketed under the trade names Avicel^{®} RC-501, Avicel^{®} RC-581, Avicel^{®} RC-591, and Avicel^{®} CL-611.

Suitable wetting agents are selected from the group comprising anionic, cationic, non-ionic, or zwitterionic surfactants, and combinations thereof. Suitable examples of wetting agents are sodium lauryl sulphate; cetrimide; polyethylene glycols such as PEG 400; polysorbates such as Tween^{®} 20, Tween^{®} 40, Tween^{®} 60, and Tween^{®} 80; polyoxyethylene-polyoxypropylene block copolymers such as poloxamers; polyglycerin fatty acid esters such as decaglyceryl monolaurate and decaglyceryl monomyristate; sorbitan fatty acid esters such as sorbitan monostearate; polyoxyethylene sorbitan fatty acid esters such as polyoxyethylene sorbitan monooleate; polyethylene glycol fatty acid esters such as polyoxyethylene monostearate; polyoxyethylene alkyl ethers such as polyoxyethylene lauryl ether; polyoxyethylene castor oil; and mixtures thereof.

The topical aqueous spray compositions of the present invention are non-foaming.

The topical aqueous spray compositions of the present invention are preservative-free.

The topical aqueous spray compositions of the present invention have a pH ranging from about 3 to about 8.

The dispensing system for delivering the pressurized composition of the present invention comprises a container and a valve-actuator assembly. The valve-actuator assembly may comprise of a valve, a spring, a gasket, a dip tube, an actuator, and a dust cap. Various types of valves, such as continuous spray valves and metering valves, can be used. A metered valve dispenses a metered quantity of formulation with each actuation of the actuator. The metered quantity avoids under-dosing or overdosing that may lead to undesirable side effects. A dust cap is fitted onto the container to shield the contents of the container from the outside environment.

The dispensing system for delivering the non-pressurized composition of the present invention comprises of a container and a pump-actuator assembly.The pump-actuator assembly may comprise of a spring, a gasket, a dip tube, a pump dispenser, a chamber, a dust cap, and an actuator. The pump dispenser dispenses the composition through a dip tube into a chamber. The composition is then dispensed through the orifice of the actuator in the form of a substantially uniform spray or fine droplets. The pump-actuator assembly may comprise of a metered pump. The metered pump dispenses a metered quantity with each actuation of the actuator. The metered quantity can avoid under-dosing or overdosing that may lead to undesirable side effects. A dust cap is fitted onto the container to shield the contents of the container from the outside environment.

Containers can be made up from metal such as stainless steel or aluminum, plastic, or glass. The plastic container can be made up of high density polyethylene (HDPE), medium density polyethylene, or low density polyethylene. The containers made up from metals can be further coated with an inert inner lining of epoxy-phenolic resins, epoxy-ureaformaldehyde resins, polytetrafluoroethylene, perfluoroethylenepropylene, perfluoroalkoxyalkane, ethylene tetrafluoroethylene, polyvinylidene fluoride, chlorinated ethylene tetrafluoroethylene, or another coating treatment that creates a barrier to chemical interaction between the composition and the container.

The amount of halobetasol depends upon the purpose for which the composition is to be applied. For example, the dosage and frequency of application can vary depending upon the type and severity of the topical condition.

The following examples represent various embodiments according to the present invention. The examples are given solely for the purpose of illustration and are not to be construed as limitations of the present invention, as many variations thereof are possible without departing from the spirit and scope of the invention.

### EXAMPLES

### Example 1

| **Ingredients** | **Quantity (%w/w)** |
|---|---|
| Halobetasol propionate | 0.05 |
| Polyethylene glycol | 20.00 |
| Citric acid | 0.04 |
| Ethyl alcohol | 40.00 |
| Water | 39.91 |

### Procedure:

1. Polyethylene glycol is mixed with ethyl alcohol to form a solution.
2. Halobetasol propionate is dissolved in the solution of step 1.
3. Citric acid is separately dissolved in a portion of water to form a solution.
4. The solution of step 3 is added into the solution of step 2 with stirring to obtain a solution.
5. The remaining portion of the water is added to the solution of step 4 with stirring.
6. The solution of step 5 is filled into an HDPE or glass container, and the container is fitted with a metered pump assembly.

### Example 2

| **Ingredients** | **Quantity (%w/w)** |
|---|---|
| Halobetasol propionate | 0.05 |
| Polyethylene glycol | 40.00 |
| Caprylic and capric triglycerides (Crodamol™ GTCC) | 20.00 |
| Tween^{®} 20 | 2.00 |
| Citric acid | 0.02 |
| Water | 37.93 |

### Procedure:

1. Halobetasol propionate is dissolved in caprylic and capric triglycerides.
2. Polyethylene glycol is mixed with a portion of water to form a solution.
3. Tween^{®} 20is added to the solution of step 2 with stirring.
4. Citric acid is added to the solution of step 3 with stirring.
5. The solution of step 4 is added into the solution of step 1 with stirring to obtain an emulsion.
6. The remaining portion of the water is added to the emulsion of step 5 with stirring.
7. The emulsion of step 6 is filled into an HDPE or glass container, and the container is fitted with a metered pump assembly.

### Example 3

| **Ingredients** | **Quantity (%w/w)** |
|---|---|
| Halobetasol propionate | 0.05 |
| Polyethylene glycol | 15.00 |
| Citric acid | 0.04 |
| Ethyl alcohol | 35.00 |
| Water | 34.91 |
| Dimethyl ether | 15.00 |

### Procedure:

1. Polyethylene glycol is mixed with ethyl alcohol to form a solution.
2. Halobetasol propionate is dissolved in the solution of step 1.
3. Citric acid is separately dissolved in a portion of water to form a solution.
4. The solution of step 3 is added to the solution of step 2 with stirring to obtain a solution.
5. The remaining portion of the water is added to the solution of step 4 with stirring.
6. The solution of step 5 is filled into an aluminum can, and the canister is crimped with a valve.
7. Dimethyl ether is filled into the can of step 6.
8. An actuator and overcap are placed over the can of step 7.

### Example 4

| **Ingredients** | **Quantity (%w/w)** |
|---|---|
| Halobetasol propionate | 0.05 |
| Polyethylene glycol | 10.00 |
| Citric acid | 0.04 |
| Ethyl alcohol | 30.00 |
| Water | 19.91 |
| Liquefied petroleum gas | 40.00 |

### Procedure:

1. Polyethylene glycol is mixed with ethyl alcohol to form a solution.
2. Halobetasol propionate is dissolved in the solution of step 1.
3. Citric acid is separately dissolved in a portion of water to form a solution.
4. The solution of step 3 is added to the solution of step 2 with stirring to obtain a solution.
5. The remaining portion of the water is added to the solution of step 4 with stirring.
6. The solution of step 5 is filled into an aluminum can, and the canister is crimped with a valve.
7. Liquefied petroleum gas is filled into the can.
8. An actuator and overcap are placed over the can of step 7.

### Example 5

| **Ingredients** | **Quantity (%w/w)** |
|---|---|
| Halobetasol propionate | 0.05 |
| Polyethylene glycol | 35.00 |
| Caprylic and capric triglycerides (Crodamol™ GTCC) | 20.00 |
| Tween^{®} 20 | 2.00 |
| Citric acid | 0.02 |
| Water | 27.93 |
| Dimethyl ether | 15.00 |

### Procedure:

1. Halobetasol propionate is dissolved in caprylic and capric triglycerides.
2. Polyethylene glycol is mixed with a portion of water to form a solution.
3. Tween^{®} 20 is added to the solution of step 2 with stirring.
4. Citric acid is added to the solution of step 3 with stirring.
5. The solution of step 4 is added to the solution of step 1 with stirring
6. The remaining portion of the water is added to the mixture of step 5 to form an emulsion.
9. The emulsion of step 6 is filled into an aluminum can, and the canister is crimped with a valve.
10. Dimethyl ether is filled into the can of step 9.
11. An actuator and overcap are placed over the can of step 10.

## Claims

1. A topical aqueous spray composition comprising:
about 0.01% w/w to about 0.5% w/w of halobetasol based on the total weight of the composition;
one or more water-miscible co-solvents; and
water , wherein the composition is placed in and/or is capable of releasing from a dispensing system for spraying said composition.

2. The topical aqueous spray composition of claim 1, wherein the composition comprises about 0.05% w/w of halobetasol based on the total weight of the composition.

3. The topical aqueous spray composition of claim 2, wherein the halobetasol is halobetasol propionate.

4. The topical aqueous spray composition of claim 1, wherein the water-miscible co-solvent is selected from group consisting of alcohols and glycols including ethyl alcohol, isopropyl alcohol, propylene glycol, butanediol, pentanediol, hexanediol, triethylene glycol, tetraethylene glycol, dipropylene glycol, and dibutylene glycol; glycerin; dimethyl isosorbide; tetrahydrofurfuryl alcohol polyethylene glycol ether; N-methyl-2-pyrrolidone; 1-methyl-2-pyrrolidinone; dimethylsulfoxide; dimethylacetamide; lactic acid; glycolic acid; methylene chloride; methyl-ethyl-ketone; ethyl acetate; methylene dimethyl ether; polyethylene glycols such as polyethylene glycol 400; and mixtures thereof.

5. The topical aqueous spray composition of claim 4, wherein the water-miscible co-solvent is ethyl alcohol.

6. The topical aqueous spray compositions of claim 1, wherein the composition is an emulsion.

7. The topical aqueous spray composition of claim 1, wherein the composition further comprises an emulsifier.

8. The topical aqueous spray composition of claim 7, wherein the water-immiscible co-solvent is selected from group consisting of fatty acid esters including isopropyl myristate and isopropyl palmitate; light and heavy liquid paraffins; fatty acid triglycerides including mixtures of caprylic and capric triglycerides (Crodamol™ GTCC, Miglyol^{®}, Captex^{®}, Labrafac^{®} Lipophile WL); mixtures of mono-, di-, and/or triglycerides; waxes; hydrogenated vegetable oils; and mixtures thereof.

9. The topical composition of claim 8, wherein the emulsifier is selected from the group consisting of sucrose esters; polymers, including poloxamers; polyysorbates, including Tween^{®} 20, Tween^{®} 40, Tween^{®} 60, and Tween^{®} 80; polyoxyethylene fatty acid esters, including Myrj^{®} 45, Myrj^{®} 49, and Myrj^{®} 59; poly(oxyethylene)alkylyl ethers including poly(oxyethylene)cetyl ether, poly(oxyethylene)palmityl ether, polyethylene oxide hexadecyl ethers such as Brij^{®} 58, and polyethylene glycol cetyl ethers such as Brij^{®} 38, Brij^{®} 52, Brij^{®} 56, and Brij^{®} W1; partial esters of sorbitol and sorbitol anhydrides including sorbitan monolaurate and sorbitan monolaurate-mono or diglycerides; alkyl isethionates; alkyl and alkyl ether sulfates and salts thereof; alkyl and alkyl ether phosphates and salts thereof; alkyl methyl taurates; soaps (e.g., alkali metal salts, sodium salts, and potassium salts of fatty acids); alkylimino acetates; imino-dialkanoates and aminoalkanoates; imidazolinium and ammonium derivatives; betaines; sultaines; hydroxysultaines; alkyl sarcosinates; alkanoyl sarcosinates, sorbitan esters, including Arlacel^{®} 20 and Arlacel^{®} 40; polyethylene glycol ethers of stearic acid including steareth-2, steareth-4, and steareth-6; polyethylene glycol ethers of stearyl alcohol (steareth-21); silicone emulsifying agents such as dimethicone copolyols; polyoxyethylene sorbitan fatty acid esters; non-solid fatty substances at room temperature, including sesame oil, sweet almond oil, apricot stone oil, sunflower oil, octoxyglyceryl palmitate (or 2-ethylhexyl glyceryl ether palmitate), octoxyglyceryl behenate (or 2-ethylhexyl glyceryl ether behenate), and dioctyl adipate; PEG-20 hexadecenyl succinate; PEG-15 stearyl ether; disodium cocoamphodiacetate; oxyethylenated glyceryl cocoate (7 EO); ricinoleic monoethanolamide monosulfosuccinate salts; oxyethylenated hydrogenated ricinoleic triglyceridesincluding Cremophor^{®} RH 60 (polyoxyl 60 hydrogenated castor oil) and Cremophor^{®} RH 40 (polyoxyl 40 hydrogenated castor oil); and mixtures thereof.

10. The topical aqueous spray composition of claim 1, wherein the composition further comprises an emollient.

11. The topical aqueous spray composition of claim 10, wherein the emollient is selected from the group consisting of fatty acid triglycerides including mixtures of caprylic and capric triglycerides (*e.g.,*Crodamol™ GTCC, Miglyol^{®}, Captex^{®}, Labrafac^{®} Lipophile WL), palmitic triglyceride, oleic triglyceride, caprylic triglyceride, capric triglyceride, and linoleic triglyceride; fatty acid esters including isopropyl myristate, isopropyl palmitate, dibutyl adipate, and dibutyl phthalate; polyhydric alcohols including propylene glycol, butylene glycol, polyethylene glycol, glycerol, and sorbitol; fatty acids including oleic acid and stearic acid; oils such as mineral oil, lanolin oil, coconut oil, cocoa butter, olive oil, jojoba oil, and castor oil; cyclomethicone; hydrogenated lanolin; waxes; lecithin; and mixtures thereof.

12. The topical aqueous spray composition of claim 11, wherein the composition further comprises a propellant.

13. The topical aqueous spray composition of claim 12, wherein the propellant is selected from the group consisting of propane, butane, isobutane, pentane, isopentane, cyclopropane, 1,1,1,2-tetrafluoroethane, 1,1,1,2,3,3,3-heptafluoropropane, 1,1-difluoroethane, 1,1,1,3,3,3-hexafluoropropane, dimethyl ether, fluorocarbon gas, liquefied petroleum gas, nitrogen, carbon dioxide, nitrous oxide, and mixtures thereof.

14. A process for the preparation of a topical aqueous spray composition of halobetasol of claim 1, wherein the process comprises the steps of:
(a) dissolving halobetasol in a water-miscible co-solvent to form a solution;
(b) mixing the solution of step (a) with water; and
(c) dispensing the solution of step (b) in a dispensing system.

15. A process for the preparation of a topical aqueous spray composition of halobetasol of claim 7, wherein the process comprises the steps of:
(a) dissolving halobetasol in a water-immiscible co-solvent to form a solution;
(b) dissolving an emulsifier in water to form a solution;
(c) mixing the solution of step (a) with the solution of step (b) to form an emulsion; and
(d) dispensing the emulsion of step (c) in a dispensing system.

16. A topical aqueous spray composition of halobetasol of claim1 for use in treating a topical skin condition.

17. The topical aqueous spray composition of claim 16, wherein the condition is one or more conditions selected from the group consisting of corticosteroid-responsive dermatoses, including psoriasis, atopic dermatitis, eczema, rosacea, acne vulgaris, pruritis, seborrhea, skin cancers, and inflammation.

18. The topical aqueous spray composition of claim 17, wherein the condition is a corticosteroid-responsive dermatosis.

19. The topical aqueous spray composition of claim 18, which comprises co-administrable additional drug(s) used to treat the topical skin condition.
